(19) Europäisches Patentamt
European Patent Office
Office européen des brevets

(11) EP 2 560 611 B1

(12) EUROPEAN PATENT SPECIFICATION

(45) Date of publication and mention
of the grant of the patent:
**03.01.2018 Bulletin 2018/01**

(21) Application number: **11719211.2**

(22) Date of filing: **19.04.2011**

(51) Int Cl.:
*A61K 9/00* (2006.01)

(86) International application number:
**PCT/EP2011/056227**

(87) International publication number:
**WO 2011/131663 (27.10.2011 Gazette 2011/43)**

(54) **"PROCESS FOR PROVIDING PARTICLES WITH REDUCED ELECTROSTATIC CHARGES"**

VERFAHREN ZUR BEREITSTELLUNG VON PARTIKELN MIT REDUZIERTEN ELEKTROSTATISCHEN LADUNGEN

PROCÉDÉ DE PRÉPARATION DE PARTICULES AYANT DES CHARGES ÉLECTROSTATIQUES RÉDUITES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.04.2010 EP 10160565**

(43) Date of publication of application:
**27.02.2013 Bulletin 2013/09**

(73) Proprietor: **Chiesi Farmaceutici S.p.A.**
**43100 Parma (IT)**

(72) Inventors:
• **COCCONI, Daniela**
**I-43100 Parma (IT)**
• **MUSA, Rossella**
**I-43100 Parma (IT)**

(74) Representative: **Minoja, Fabrizio**
**Bianchetti Bracco Minoja S.r.l.**
**Via Plinio, 63**
**20129 Milano (IT)**

(56) References cited:
**WO-A2-2005/104712        US-A1- 2003 180 227**
**US-A1- 2004 202 616**

• **GUCHARDI ET AL: "Influence of fine lactose and magnesium stearate on low dose dry powder inhaler formulations", INTERNATIONAL JOURNAL OF PHARMACEUTICS, ELSEVIER BV, NL LNKD- DOI:10.1016/J.IJPHARM.2007.06.041, vol. 348, no. 1-2, 19 December 2007 (2007-12-19), pages 10-17, XP022393884, ISSN: 0378-5173**
• **ELAJNAF A ET AL: "Electrostatic characterisation of inhaled powders: Effect of contact surface and relative humidity", EUROPEAN JOURNAL OF PHARMACEUTICAL SCIENCES, ELSEVIER, AMSTERDAM, NL LNKD-DOI:10.1016/J.EJPS.2006.07.006, vol. 29, no. 5, 1 December 2006 (2006-12-01), pages 375-384, XP025137181, ISSN: 0928-0987 [retrieved on 2006-12-01]**
• **CHAN ET AL: "Dry powder aerosol drug delivery-Opportunities for colloid and surface scientists", COLLOIDS AND SURFACES. A, PHYSICACHEMICAL AND ENGINEERING ASPECTS, ELSEVIER, AMSTERDAM, NL LNKD-DOI:10.1016/J.COLSURFA.2005.10.091, vol. 284-285, 15 August 2006 (2006-08-15), pages 50-55, XP025136551, ISSN: 0927-7757 [retrieved on 2006-08-15]**
• **KEAT THENG CHOW ET AL: "Investigation of Electrostatic Behavior of a Lactose Carrier for Dry Powder Inhalers", PHARMACEUTICAL RESEARCH, KLUWER ACADEMIC PUBLISHERS-PLENUM PUBLISHERS, NL LNKD-DOI:10.1007/S11095-008-9651-Y, vol. 25, no. 12, 26 June 2008 (2008-06-26) , pages 2822-2834, XP019647910, ISSN: 1573-904X**

- PHILIP CHI LIP KWOK ET AL: "Effect of Relative Humidity on the Electrostatic Charge Properties of Dry Powder Inhaler Aerosols", PHARMACEUTICAL RESEARCH, KLUWER ACADEMIC PUBLISHERS-PLENUM PUBLISHERS, NL, vol. 25, no. 2, 10 July 2007 (2007-07-10), pages 277-288, XP019579449, ISSN: 1573-904X

- PAUL M YOUNG ET AL: "Influence of Humidity on the Electrostatic Charge and Aerosol Performance of Dry Powder Inhaler Carrier based Systems", PHARMACEUTICAL RESEARCH, KLUWER ACADEMIC PUBLISHERS-PLENUM PUBLISHERS, NL LNKD-DOI:10.1007/S11095-006-9218-8, vol. 24, no. 5, 22 March 2007 (2007-03-22) , pages 963-970, XP019507204, ISSN: 1573-904X

**Description**

**TECHNICAL FIELD**

The present invention concerns a process for preparing dry powder formulations for inhalation. In particular the present invention concerns a process for preparing carrier particles for dry powder formulations having reduced electrostatic charges.

**BACKGROUND OF THE INVENTION**

Dry powder inhalation (DPI) drug therapy has been used for many years to treat respiratory conditions such as asthma, chronic obstructive pulmonary disease (COPD), and allergic rhinitis.

Drugs intended for inhalation as dry powders should be used in the form of micronised particles which are generally obtained by milling or through other techniques such as spray-drying.

Dry powder formulations intended for inhalation are typically prepared by mixing the micronised drug with coarse carrier particles, giving rise to ordered mixture where the micronised active particles adhere to the surface of the carrier particles whilst in the inhaler device.

The carrier makes the micronised powder less cohesive and improves its flowability, making the handling of the powder during the manufacturing process (pouring, filling etc.) easier.

However, it is known that dry powders tend to become electrostatically charged. Triboelectrification in pharmaceutical powders is a very complicated and not-well understood process although it has been shown to be influenced by many factors.

During the various manufacturing operations (milling, mixing, transport and filling), powders accumulate electrostatic charges from inter-particulate collisions and contact with solid surfaces (e.g. vessel walls).

This process of both contact- and tribology- induced electrification has been identified in the mechanisms of drug loss via segregation, adhesion and agglomeration formation. Furthermore, the more energy involved during a process, the greater the propensity for the materials to build-up significant levels of electrostatic charges.

The following table presents some typical charge values for different manufacturing operations of a dry powder formulation.

*Typical charge generation during powder processing operations Reference: Code of practice for control of undesirable static electricity, BS 5958 (British Standards Institution, London, 1991)*

| Operation | Mass Charge Density ($\mu$C/Kg) |
|---|---|
| Sieving | $10^{-3}$ - $10^{-6}$ |
| Pouring | $10^{-1}$ - $10^{-3}$ |
| Feed transfer | $1$ - $10^{-2}$ |
| Micronizing | $10^{2}$ - $10^{-1}$ |
| Pneumatic Conveying | $10^{3}$ - $10^{-1}$ |

The net electrostatic charge of a powder blend is highly dependant on the frequency of particle-substrate and particle-particle collisions during manufacturing, which can invariably lead to a net charge on the powder sample that may be positive, negative or both.

WO 01/78693 and WO 01/78695 disclose dry powder formulations comprising as a carrier, a fraction of coarse particles and a fraction made of fine particles and an additive such as magnesium stearate or leucine, and processes of preparation thereof.

US 2004/02022616 discloses the use of magnesium stearate to improve the moisture resistance of dry powder formulations for inhalation.

Guchardi R et al Int J Pharm 2008, 348, 10-17 investigate the behaviour of dry powders for inhalation depending on the amount of fine lactose particles and the presence of magnesium stearate.

Said formulations can be produced in a simple way, are chemically and physically stable and provided with good inhalatory performances.

However, said documents do not provide any information regarding the electrostatic charges.

On the other hand, the reduction of electrostatic chargeability may improve the flow properties during the operations of the manufacture process (sieving, pouring) and during the filling of the inhaler.

This in turn would lead to an improved homogeneity of the active ingredient in the formulation, and hence to an improved reproducibility and accuracy of the delivered dose and the fine particle dose.

In view of the above considerations, it would be highly advantageous to provide a process for preparing powder formulations such as those described in WO 01/78693 and WO 01/78695 able to reduce electrostatic charges, and hence improve their performance characteristics.

## SUMMARY OF THE INVENTION

The invention is directed to a process for preparing a carrier particles for dry powder formulation for inhalation comprising i) a fraction of co-micronised particles made of a mixture of an excipient and an additive, the mixture having a MMD lower than 20 micron; ii) a fraction of coarse excipient particles having a MMD equal to or higher than 80 micron, said process comprising the following steps:

a) co-micronising the excipient particles and additive particles;
b) adding and mixing the obtained co-micronised particles with the coarse excipient particles; characterised in that the co-micronised particles of step a) are first conditioned by exposure to a relative humidity of 50-75% at room temperature for a time comprised between 24 and 60 hours, wherein room temperature means 22 $\pm$ 2°C.

In a second aspect, the invention is directed to a process for preparing a dry powder formulation for inhalation comprising the step of mixing the above carrier particles with one or more active ingredients.

The invention also discloses mixture of co-micronised particles made of an excipient and an additive for use in a dry powder formulation for inhalation, said mixture having a mass charge density comprised between -9 x$10^{-10}$ and -5 x $10^{-8}$ nC/g, said mixture being obtainable by a process which comprises conditioning by exposure to a relative humidity of 50-75% at room temperature for a time comprised between 24 and 60 hours.

The invention also discloses a dry powder formulation for inhalation comprising the aforementioned mixture of co-micronised particles and one or more active ingredients.

The invention also discloses a dry powder inhaler filled with the above dry powder formulation.

The invention also discloses the use of the disclosed mixture of co-micronised particles for the preparation of a medicament for the prophylaxis and/or treatment of a pulmonary disease, such as asthma or chronic obstructive pulmonary disease (COPD).

## DEFINITIONS

The terms "active drug", "active ingredient", "active" and "active agent", "active compound" and "therapeutic agent" are used as synonymous.

The term "hygroscopic" refers to an active compound that ever completely dries in contact with air having a moisture content of >0% relative humidity, but always contains a certain amount of absorptively bound water (H. Sucker, P. Fuchs and P. Speiser: Pharmaceutical Technology, Georg Thieme Verlag, Stuttgart, New York, 2nd edition 1991, page 85).

The term "hydrophilic" refers to an active ingredient that can easily be wetted by water.

The term "conditioning" means an exposure of the powder placed in a suitable container to a combination of temperature and relative humidity conditions kept under control.

By "therapeutically effective dose" it is meant the quantity of active ingredient administered at one time by inhalation upon actuation of the inhaler.

For "actuation" it is meant the release of active ingredient from the device by a single activation (e.g. mechanical or breath).

The term "low-dosage strength active ingredient" means an active ingredient to be delivered using a dry powder inhaler (DPI) device whose the dose delivered after each actuation of the inhaler is equal to or lower than 12 $\mu$g, preferably equal to or lower than 6 $\mu$g, more preferably equal to or lower than 4 $\mu$g, even more preferably lower than 2 $\mu$g.

In general terms, the particle size of particles is quantified by measuring a characteristic equivalent sphere diameter, known as volume diameter, by laser diffraction.

The particle size can also be quantified by measuring the mass diameter by means of suitable known instrument such as, for instance, the sieve analyser.

The volume diameter (VD) is related to the mass diameter (MD) by the density of the particles (assuming a size independent density for the particles).

In the present application, the particle size is expressed in terms of mass diameter and the particle size distribution is expressed in terms of the mass median diameter (MMD) which corresponds to the diameter of 50 percent by weight of the particles [d(0.5)], and, optionally, also in terms of mass diameter in micron of 10% and 90% of the particles, respectively [d(0.1) and d(0.9)].

The term "hard pellets" refers to spherical or semispherical units whose core is made of coarse excipient particles.

The term "spheronisation" refers to the process of rounding off of the particles which occurs during the treatment.

The term "fluidisation" refers to the property of a carrier based DPI formulation of being "fluidise" i.e. of being easily transported in the air stream during the aerosol formation. Said property is dependant on the resistance (cohesivity) of the mixture.

The term "good flowability" refers to a formulation that is easy handled during the manufacturing process and is able to ensure an accurate and reproducible delivering of the therapeutically effective dose.

Flow characteristics can be evaluated by different tests such as angle of repose, Carr's index, Hausner ratio or flow rate through an orifice.

In the context of the present application the flow properties were tested by measuring the flow rate through an orifice according to the method described in the European Pharmacopeia (Eur. Ph.).

The expression "good homogeneity" refers to a formulation wherein, upon mixing, the uniformity of distribution of the active ingredient, expressed as coefficient of variation (CV) also known as relative standard deviation (RSD), is less than 2.5%, preferably equal to or less than 1.5%.

The expression "respirable fraction" refers to an index of the percentage of active particles which would reach the deep lungs in a patient.

The respirable fraction, also termed fine particle fraction, is evaluated using a suitable *in vitro* apparata such as Multistage Cascade Impactor or Multi Stage Liquid Impinger (MLSI) according to procedures reported in common Pharmacopoeias.

It is calculated by the ratio between the respirable dose and the delivered dose.

The delivered dose is calculated from the cumulative deposition in the apparatus, while the respirable dose (fine particle dose) is calculated from the deposition on Stages 3 (S3) to filter (AF) corresponding to particles $\leq$ 4.7 microns.

The "delivered dose" is the percentage of the metered dose of medication delivered to the lungs of a patient. For low dosage strength active ingredients such as formoterol, said percentage is theoretically considered of 75%.

The expression "accurate" with reference to the dose of the active ingredient refers to the variation between the theoretical delivered dose and the actual delivered dose. The minor the variation, the higher is the accuracy. For a low dosage strength active ingredient, a good accuracy is given by a variation equal to lower than $\pm$ 5%, preferably lower $\pm$ 2.5%.

The term "reproducibility" refers to the degree of closeness of the measurements and is expressed by the coefficient of variation (CV) also known as relative standard deviation (RSD).

The minor the CV, the higher is the reproducibility. A good reproducibility is given by a CV of less than 10%, preferably less than 5%, more preferably less than 2.5%.

The term "coating" refers to the covering of the surface of the excipient particles by forming a thin film of magnesium stearate around said particles.

## FIGURES

Figure 1 - Surface energy of micronised particles and reference materials as determined by IGC.
Figure 2 - A comparison of the OD stretching band in the FT-Raman spectra of the samples #1, #2, #3, #4 and #7.

## DETAILED DESCRIPTION OF THE INVENTION

The invention is directed to a process for preparing carrier particles for dry powder formulation for inhalation comprising i) a fraction of co-micronized particles made of a mixture of an excipient and an additive, ii) a fraction of coarse excipient particles, and one or more active ingredients, said process comprising the following steps:

a) co-micronising the excipient particles and additive particles;
b) adding and mixing the obtained co-micronised particles with the coarse excipient particles; characterized in that the co-micronised particles of step a) are first conditioned by exposure under particular conditions.

As a result of the conditioning step, charge acquisition of the co-micronised particles, and hence of all the carrier particles, is reduced. The corresponding powder formulations comprising said carrier particles exhibit better flow properties than those comprising a carrier comprising the not-conditioned co-micronised particles.

Moreover, the formulations comprising carrier particles subjected by the process of the invention show an improved homogeneity of the active ingredient, as well as better accuracy of the delivered dose and better reproducibility of the fine particle dose than the formulation not subjected to conditioning.

Even when it comprises a low dosage strength active ingredient, for the formulation comprising carrier particles subjected by the process of the invention, the accuracy of the delivered dose is usually better than $\pm$ 5%, preferably than $\pm$ 2.5%.

Surprisingly, upon conditioning, the fraction of co-micronised particles also shows a reduction in the inter-particles cohesive interactions as suggested by the decrease in the basic flow energy and the energy required overcoming the resistance of the material to fluidize as measured by the fluidization energy.

As a consequence of all these advantages, the respirable fraction of the relevant formulation as well turned out to be slightly improved.

Upon conditioning, the amorphous material generated during the micronisation step is also significantly diminished, suggesting that said step induces an effective re-crystallization of the excipient particles.

On the other hand, the identified conditions of exposure do not affect in a significant way the particle size and the water content of the co-micronised particles.

The latter aspect is beneficial for the stability of the active ingredient(s) in the relevant formulation as it is known that an increase in moisture sorption could affect their physico-chemical stability, in particular of hygroscopic and/or hydrophilic active ingredients.

The co-micronized particles must be conditioned by exposure at room temperature to a relative humidity comprised between 50 and 75% for a time comprised between 24 and 60 hours.

The room temperature corresponds to a temperature of 22 $\pm$ 2°C, preferably $\pm$ 1°C.

Advantageously the exposure is carried out at a relative humidity of between 55 and 70% for a time comprised between 24 and 48 hours, more preferably for 48 hours. In a preferred embodiment, said exposure is carried out at a relative humidity of 55% for 24 hours, while in other preferred embodiment, the exposure is carried out at a relative humidity of 75% for 24 hours. In further preferred embodiments, the exposure is carried out at a relative humidity of at least 55% for 48 hours as it has been observed that the reduction of the surface energy of the co-micronised particles is greater starting from said value of relative humidity and for longer times.

The values of relative humidity could vary of $\pm$ 5%.

Without being limited by the theory, it can be hypothesized that the higher the surface energy, the higher is the reactivity of material and hence the higher is the probability of the formation of electrostatic charges.

Advantageously, the fine and coarse excipient particles may be constituted of any pharmacologically acceptable inert material or combination thereof; preferred excipients are those made of crystalline sugars, in particular lactose; the most preferred are those made of $\alpha$-lactose monohydrate.

Preferably, the coarse excipient particles and the fine excipient particles are constituted of the same physiologically acceptable pharmacologically-inert material.

The fraction of co-micronised particles made of a mixture of an excipient and an additive must have a MMD lower than 20 micron, advantageously equal to or lower than 15 micron, preferably equal to lower than 10 micron, even more preferably equal to or lower than 6 micron.

Advantageously, the mass diameter of 90% of the particles is lower than 35 micron, more advantageously lower than 25 micron, preferably lower than 15 micron, even more preferably lower than 10 micron.

The ratio between the excipient and the additive within the fraction of micronised particles will vary depending on the composition of the formulation and the nature and properties of the additive material.

Advantageously, said fraction of co-micronised particles is composed of 90 to 99.5% by weight of the excipient and 0.5 to 10% by weight of the additive material, preferably of 95 to 99% of the excipient, and 1 to 5% of the additive. A preferred ratio is 98% of the excipient and 2% of the additive.

Advantageously, the additive material may include or consist of one or more lubricant selected from the group consisting of stearic acid and salts thereof such as magnesium stearate, sodium lauryl sulphate, sodium stearyl fumarate, stearyl alcohol, sucrose monopalmitate.

Preferably, the lubricant is magnesium stearate.

Alternatively, the additive material may be an anti-adherent material such as an amino acid, preferably selected from the group consisting of leucine, isoleucine, lysine, valine, methionine, phenylalanine. The additive may be a salt of a derivative of an amino acid, for example aspartame or acesulfame K.

The additive material may also include or consist of one or more water soluble surface active materials, for example lecithin, in particular soya lecithin.

Other possible additive materials include talc, titanium dioxide, aluminium dioxide, and silicon dioxide.

Advantageously, at least 90% by weight of the additive particles has a starting mass diameter of not more than 35 micron and a MMD of not more than 15 micron, preferably not more than 10 micron.

The excipient particles and additive particles constituting the fraction of micronised particles are co-micronised by milling, advantageously in a ball mill. In some cases, co-micronisation for at least two hours may be found advantageous, although it will be appreciated that the time of treatment will generally depend on the starting particle size of the excipient particles and the desired size reduction to be obtained.

In a preferred embodiment of the invention the particles are co-micronised starting from excipient particles having a mass diameter less than 250 micron and an additive having a mass diameter less than 35 micron using a jet mill, preferably in inert atmosphere, for example under nitrogen.

As an example, alpha-lactose monohydrate commercially available such as Meggle D 30 or Spherolac 100 (Meggle, Wasserburg, Germany) could be used as starting excipient.

The coarse excipient particles of the process of the invention must have a MMD of at least 80 micron, more advantageously greater that 90 micron, preferably greater than 100 micron, more preferably greater than 175 micron.

Advantageously, all the coarse particles have a mass diameter in the range 50-1000 micron, preferably comprised between 60 and 500 micron.

In certain embodiments of the invention, the mass diameter of said coarse particles might be comprised between 80 and 200 micron, preferably between 90 and 150 micron, while in another embodiment, the mass diameter might be comprised between 200 and 400 micron, preferably between 210 and 355 micron.

In general, the person skilled in the art will select the most proper size of the coarse excipient particles by sieving, using a proper classifier.

When the mass diameter of the coarse particles is comprised between 200 and 400 micron, the coarse excipient particles have preferably a relatively highly fissured surface, that is, on which there are clefts and valleys and other recessed regions, referred to herein collectively as fissures. The "relatively highly fissured" coarse particles can be defined in terms of fissure index or rugosity coefficient as described in WO 01/78695 and WO 01/78693, incorporated herein by reference, and they can be characterized according to the description therein reported. Said coarse particles may also be characterized in terms of tapped density or total intrusion volume measured as reported in WO 01/78695.

The tapped density of said coarse particles is advantageously less than 0.8 g/cm$^3$, preferably between 0.8 and 0.5 g/cm$^3$. The total intrusion volume is of at least 0.8 cm$^3$ preferably at least 0.9 cm$^3$.

The ratio between the fraction of micronised particles and the fraction of coarse particles is comprised between 1:99 and 40:60% by weight, preferably between 2:98 and 30:70% by weight, even more preferably between 5:95 and 20:80% by weight. In a preferred embodiment, the ratio is comprised between 10:90 and 15:85% by weight.

The step of mixing the coarse excipient particles and the micronised particle fraction is typically carried out in a suitable mixer, e.g. tumbler mixers such as Turbula, rotary mixers or instant mixer such as Diosna for at least 5 minutes, preferably for at least 30 minutes, more preferably for at least two hours. In a general way, the person skilled in the art will adjust the time of mixing and the speed of rotation of the mixer to obtain homogenous mixture.

When spheronized coarse excipient particles are desired in order to obtain hard-pellets, the step of mixing will be typically carried out for at least four hours.

In a preferred embodiment, the invention is directed to a process for preparing carrier particles for dry powder formulation for inhalation comprising: i) a fraction of co-micronised particles having a MMD equal to or lower than 10 micron made of a mixture of 98 to 99% by weight of a-lactose monohydrate and 1 to 2% by weight of magnesium stearate; ii) a fraction of coarse particles made of α-lactose monohydrate, having a mass diameter comprised between 212 and 355 micron, the ratio between the co-micronised particles and the coarse particles being comprised between 10:90 and 15:85% by weight, said process comprising the following steps:

a) co-micronising the α-lactose monohydrate particles and the magnesium stearate particles;
b) adding and mixing the obtained co-micronised particles with the coarse particles; characterised in that the co-micronised particles of step a) are conditioned by exposure at room temperature at a relative humidity of between 55 and 75% for a time comprised between 24 and 48 hours.

The present invention is also directed to a process for preparing a dry powder formulation for inhalation comprising the step of mixing the carrier particles obtainable by the claimed process with one or more active ingredients.

Advantageously, at least 90% of the particles of the drug (active ingredient) have a particle size less than 10 micron, preferably less than 8 micron, more preferably less than 6 micron.

In certain embodiments of the invention, in particular when low-dosage strength active ingredients are used, no more than 50% of particles have a volume diameter lower than 1.7 micron; and at least 90% of the particles have a volume diameter lower than 8 micron.

The mixture of the carrier particles with the active ingredient particles will be prepared by mixing the components in suitable mixers like those reported above.

Optionally, when at least two active ingredients are used, one active ingredient is first mixed with a portion of the carrier particles and the resulting blend is forced through a sieve, then, the further active ingredients and the remaining part of the carrier particles are blended with the sieved mixture; and finally the resulting mixture is sieved through a sieve, and mixed again.

The skilled person shall select the mesh size of the sieve depending on the particle size of the coarse excipient particles.

The ratio between the carrier particles and the active ingredient will depend on the type of inhaler device used and the required dose.

The amount of the active ingredient shall be able to allow delivering into the lung a therapeutically effective dose.

Suitable active agents may be drugs for therapeutic and/or prophylactic use. Active agents which may be included in the formulation include those products which are usually administered orally by inhalation for the treatment of disease such a respiratory disease.

Therefore, suitable active agents include for example β2-adrenoceptor agonists such as salbutamol, terbutaline, rimiterol, fenoterol, reproterol, bitolterol, salmeterol, formoterol, clenbuterol, procaterol, broxaterol, picumeterol, carmoterol, indacaterol, milveterol mabuterol, olodaterol, vilanterol and the like; corticosteroids such as budesonide, fluticasone, in particular as propionate or furoate ester, mometasone, in particular as furoate ester, beclomethasone, in particular as 17-propionate or 17,21-dipropionate esters, ciclesonide, triamcinolone acetonide, flunisolide, zoticasone, flumoxonide, rofleponide, butixocort as propionate ester, prednisolone, prednisone, tipredane; anticholinergic bronchodilators such as, ipratropium bromide, tiotropium bromide oxitropium bromide, glycopyrronium bromide in form of (3R,2R') enantiomer or racemic mixture (3S,2R') and (3R,2S'), oxybutynin chloride, aclidinium bromide, trospium chloride, the compounds known with the codes GSK 573719 and GSK 1160274 or those described in WO 2010/015324; phosphodiesterase IV (PDE-IV) inhibitors such as filaminast, piclamilast, roflumilast or those disclosed in WO 2008/006509 and in WO 2009/018909; antihistamines; expectorants; mucolytics; cyclooxygenase inhibitors; leukotriene synthesis inhibitors; leukotriene antagonists; phospholipase-A2 inhibitors; platelet aggregating factor (PAF) antagonists.

Other active agents which may be utilized for delivery by inhalation include antiarrythmic medicaments, tranquilisers, statins, cardiac glycosides, hormones, antihypertensive medicaments, antidiabetic, antiparasitic and anticancer medicaments, sedatives and analgesic medicaments, antibiotics, antirheumatic medicaments, immunotherapies, antifungal and anti-hypotension medicaments, vaccines, antiviral medicaments, proteins, polypeptides and peptides for example peptide hormones and growth factors, polypeptides vaccines, enzymes, endorphins, lipoproteins and polypeptides involved in the blood coagulation cascade, vitamins and others, for example cell surface receptor blockers, antioxidants and free radical scavengers. Several of these compounds could be administered in the form of pharmacologically acceptable esters, acetals, salts, solvates, such as hydrates, or solvates of such esters or salts, if any. Both racemic mixtures as well as one or more optical isomers of the above compounds are within the scope of the invention.

Suitable physiologically acceptable salts include acid addition salts derived from inorganic and organic acids, for example the chloride, bromide, sulphate, phosphate, maleate, fumarate, citrate, tartrate, benzoate, 4-methoxybenzoate, 2-or 4-hydroxybenzoate, 4-chlorobenzoate, p-toluenesulphonate, methanesulphonate, ascorbate, acetate, succinate, lactate, glutarate, tricarballylate, hydroxynaphthalene-carboxylate (xinafoate) or oleate salt or solvates thereof.

Many of the above mentioned classes of pharmacologically active compounds may be administered in combination.

Formulations comprising a low dosage strength active ingredients and combinations thereof are preferred.

Formulations comprising a beta$_2$-agonist, an anti-cholinergic or a corticosteroid for inhalation, alone or in any combination thereof constitute a particular embodiment of the invention.

Preferred combinations include formoterol fumarate dihydrate/beclometasone dipropionate, vilanterol/fluticasone furoate, salmeterol xinafoate/fluticasone propionate, formoterol fumarate dehydrate/ciclesonide, formoterol fumarate dehydrate/mometasone furoate, formoterol fumarate dehydrate/budesonide, formoterol fumarate dehydrate/fluticasone propionate, formoterol fumarate dehydrate/tiotropium bromide, formoterol fumarate dihydrate/glycopyrronium bromide, and formoterol fumarate dihydrate/glycopyrronium bromide/beclometasone dipropionate, formoterol fumarate dihydrate/tiotropium bromide/beclometasone dipropionate.

The combinations comprising formoterol fumarate dihydrate, beclometasone dipropionate and optionally an anticholinergic bronchodilator such as tiotropium bromide or glycopyrronium bromide are particularly preferred.

The invention also discloses a mixture of co-micronised particles made of an excipient and an additive having a very low residual a of negative electrostatic charges, said mixture being obtainable by a process which comprises conditioning by exposure to a relative humidity of 50-75% at room temperature for a time comprised between 24 and 60 hours. The mass charge density should be comprised between $-9 \times 10^{-10}$ and $-5 \times 10^{-8}$ nC/g, preferably between $-9 \times 10^{-9}$ and $-1 \times 10^{-9}$.

The mass charge density shall be determined using a Faraday cage as described in Example 2.

The disclosed mixtures are also characterized by improved fluidisation properties as evidenced by their basic flow energy (BFE) and their fluidisation energy which are significant lower than those of the not-conditioned mixture.

The BFE is advantageously comprised between 15 and 30 mJ, preferably between 18 and 26 mJ, while the fluidization energy is advantageously comprised between 5 and 15 mJ, preferably between 8 and 12 mJ.

Upon conditioning, the amount of amorphous material is advantageously less 5% w/w, preferably less than 3% w/w, more preferably less than 2% w/w, even more preferably equal to or less than 1% w/w. The amount of amorphous material can be determined by known methods.

For instance, it can be determined as reported in Example 4 by a spectroscopy approach involving H/D exchange and FT-Raman spectroscopy. Otherwise it can be determined by dynamic vapour sorption (DVS) experiments using for example a Hiden Igasorb moisture balance or by Isothermal Gas Perfusion Calorimetry (IGPC) using for example a 2277 Thermal Activity Monitor calorimeter (TA Instrument Ltd).

In general, the amount of additive shall be not more than 10% by weight, based on the total weight of the mixture of the co-micronised particles.

However, it is thought that for most additives the amount of additive material should be not more than 5%, preferably not more than 2% or even not more than 1% by weight or not more than 0.5% based on the total weight of the mixture. In general, the amount of additive material is of at least 0.01% by weight based on the total weight of the mixture.

In one of the preferred embodiments of the invention, the excipient is a-lactose monohydrate and the additive material is magnesium stearate present in an amount comprised between 0.5 and 2%, preferably 2% by weight based on the total weight of the mixture.

The additive may form a coating around the surface of the excipient particles, or may form a discontinuous covering as reported in WO 96/23485.

If magnesium stearate is used, the additive coats the surface of the excipient particles in such a way that the extent of the surface coating is at least of 5%, preferably more than 10%, more preferably more than 15%, even more preferably equal to or more than 35%.

The extent of surface coating, which indicates the percentage of the total surface of the excipient particles coated by magnesium stearate, may be determined by water contact angle measurement and then applying the equation known in the literature as Cassie and Baxter, cited at page 338 of Colombo I et al Il Farmaco 1984, 39(10), 328-341 and reported below.

$$\cos\vartheta_{\text{mixture}} = f_{\text{MgSt}}\cos\vartheta_{\text{Mgst}} + f_{\text{lactose}}\cos\vartheta_{\text{lactose}}$$

where $f_{\text{MgSt}}$ and $f_{\text{lactore}}$ are the surface area fractions of magnesium stearate and of lactose;

$\vartheta_{\text{MgSt}}$ is the water contact angle of magnesium stearate;

$\vartheta_{\text{lactose}}$ is the water contact angle of lactose

$\vartheta_{\text{mixture}}$ are the experimental contact angle values.

For the purpose of the invention, the contact angle may be determined with methods that are essentially based on a goniometric measurement. These imply the direct observation of the angle formed between the solid substrate and the liquid under testing. It is therefore quite simple to carry out, being the only limitation related to possible bias stemming from intra-operator variability. It should be, however, underlined that this drawback can be overcome by adoption a fully automated procedure, such as a computer assisted image analysis. A particularly useful approach is the sessile or static drop method which is typically carried out by depositing a liquid drop onto the surface of the powder in form of disc obtained by compaction (compressed powder disc method).

The extent to which the magnesium stearate coats the surface of the lactose particles may also be determined by scanning electron microscopy (SEM), a well known versatile analytical technique.

Such microscopy may be equipped with an EDX analyzer (an Electron Dispersive X- ray analyzer), that can produce an image selective to certain types of atoms, for example magnesium atoms. In this manner it is possible to obtain a clear data set on the distribution of magnesium stearate on the surface of carrier particles.

SEM may alternatively be combined with IR or Raman spectroscopy for determining the extent of coating, according to known procedures.

Another analytical technique that may advantageously be used is X-ray photoelectron spectroscopy (XPS), by which it has been possible to calculate both the extent of coating and the depth of the magnesium sterate film around the lactose particles.

The disclosed mixture of co-micronised particles can be used in any dry powder formulation for inhalation.

Preferably, it is used in dry powder formulations further comprising the coarse excipient particles mentioned above and one or more active ingredients selected from the classes mentioned above.

Said dry powder formulations may be utilized with any dry powder inhaler.

Dry powder inhalers can be divided into two basic types:

i) single dose inhalers, for the administration of single subdivided doses of the active compound; each single dose is usually filled in a capsule;

ii) multidose inhalers pre-loaded with quantities of active principles sufficient for longer treatment cycles.

Said dry powder formulation for inhalation is particularly suitable for multidose dry powder inhalers comprising a reservoir from which individual therapeutic dosages can be withdrawn on demand through actuation of the device, for example that described in WO 2004/012801. Other multi-dose devices that may be used are for instance the DISKUS™ of GlaxoSmithKline, the TURBOHALER™ of AstraZeneca, TWISTHALER™ of Schering and CLICKHALER™ of Innovata. As marketed examples of single-dose devices, there may be mentioned ROTOHALER™ of GlaxoSmithKline and HANDIHALER™ of Boehringer Ingelheim.

The process of the invention is illustrated by the following examples.

## EXAMPLES

Example 1 - Preparation of the co-micronised particles made of excipient and additive

About 40 kg of co-micronised particles was prepared.

Particles of $\alpha$-lactose monohydrate having a particle size of less than 250 micron (Meggle D 30, Meggle), and magnesium stearate particles having a particle size of less than 35 micron in a ratio 98:2 percent by weight were co-micronised by milling in a jet mill operating under nitrogen to obtain the fraction of co-micronised particles.

At the end of the treatment, said co-micronized particles have a mass median diameter (MMD) of about 6 micron.

Afterwards, a part of the batch was kept separately as control and the rest was subject to conditioning at a temperature of 22 $\pm$ 1°C at different conditions of relative humidity and time reported in Table 1.

The values of relative humidity could vary of $\pm$ 5%.

All the samples were stored in polyethylene bags.

**Table 1**

| Sample | Relative humidity | Time |
|--------|-------------------|------|
| # 1 | 55% | 24 h |
| # 2 | 55% | 48 h |
| # 3 | 60% | 24 h |
| # 4 | 60% | 48 h |
| # 5 | 65% | 24 h |
| # 6 | 65% | 48 h |
| # 7 | 70% | 24 h |
| # 8 | 75% | 24 h |

Example 2 - Determination of electrostatic charges and fluidisation properties

Measurements were conducted applying the Nanoer™ technology (Nanopharm Ltd, Bath, UK).

A Faraday Pail connected to an electrometer was used to measure electrostatic charge of micronised partides. The electrometer was connected to a computer for data acquisition. 10 g of material was placed into the Faraday cage, following which the specific charge was obtained by dividing the net charge measured on the electrometer by the mass of material that entered the Faraday cage.

Micronised parties were characterized using the FT4 Powder Rheometer (Freeman Technologies, Welland, UK) to determine the resistance to aeration quantified as fluidization energy of the different powders. In each case, 10 ml of sample powder was analysed in a 25 mm bore cylinder. The samples were conditioned to remove packing history using a 23.5 mm blade that was traversed down a helical path at 20 mm/s. As the mass, volume, height and applied force experienced by the powder bed were recorded, the bulk density of the respective powders was also determined.

The results of the measurement of the electrostatic charge are reported in Table 2.

**Table 2** - **Electrostatic charge data**

| Sample | Specific charge (nC/g) $\pm$ S.D |
|--------|-----------------------------------|
| #1 | -6.7 x $10^{-9}$ $\pm$ 3.7 x $10^{-9}$ |

(continued)

| Sample | Specific charge (nC/g) $\pm$ S.D |
|---|---|
| #2 | $-3.9 \times 10^{-9} \pm 3.3 \times 10^{-9}$ |
| #5 | $-9.7 \times 10^{-9} \pm 6.9 \times 10^{-9}$ |
| #6 | $-4.8 \times 10^{-9} \pm 5.4 \times 10^{-10}$ |

The values indicate that the samples subjected to conditioning exhibit some very low residual electronegative charge, while the not conditioned sample exhibits bipolar charge.

The results in terms of Basic Flow Energy (BFE) and fluidisation energy are reported in Table 3.

**Table 3 - BFE and Fluidisation Energy data**

| Sample | Basic Flow Energy (mJ $\pm$ S.D.) | Fluidization Energy (mJ $\pm$ S.D.) |
|---|---|---|
| Not-conditioned | 25.8 (1.3) | 11.9 (1.3) |
| #1 | 22.0 (1.4) | 11.3 (1.2) |
| #2 | 19.3 (2.3) | 10.7 (1.1) |
| # 5 | 16.9 (0.3) | 6.7 (0.6) |
| # 6 | 18.0 (1.5) | 8.6 (0.5) |

Upon conditioning, there is a reduction in the cohesive interactions within the co-micronised particles. That is showed by the decrease in Basic Flow Energy (measure of flow behavior of the powder), and Fluidization Energy (energy required to overcome the resistance to fluidize).

It is possible to notice a decrease of BFE with the increase of relative humidity percentage.

Example 3 - Determination of the surface energy

The surface energies were measured by inverse gas chromatography (IGC).

All analyses were carried out using the SMS-iGC 2000 and the SMS-iGC v1.3 standard analysis suite and SMS-iGC v1.21 advanced analysis suite of macros. A flame ionisation detector (FID) was used to determine the retention times.

The samples were stored in a cold (~5°C), dry environment until run on the IGC. For all experiments, the powders were packed into a silanised glass column (300 mm long by 4 mm diameter) using the SMS Column Packing Accessory. All columns were analysed 3 times sequentially to check for irreversible chemisorption effects and equilibrium after preconditioning.

In this study the columns were pre-treated for 2 hours at 25°C and 0% RH in a helium carrier gas to condition the sample. Then, the surface energy measurements were performed at 25°C (3 times sequentially with a 2-hour conditioning between runs). All experiments were carried out at 10 sccm total flow rate of helium, and injection vapor concentration of 0.03 P/0 for all elutants.

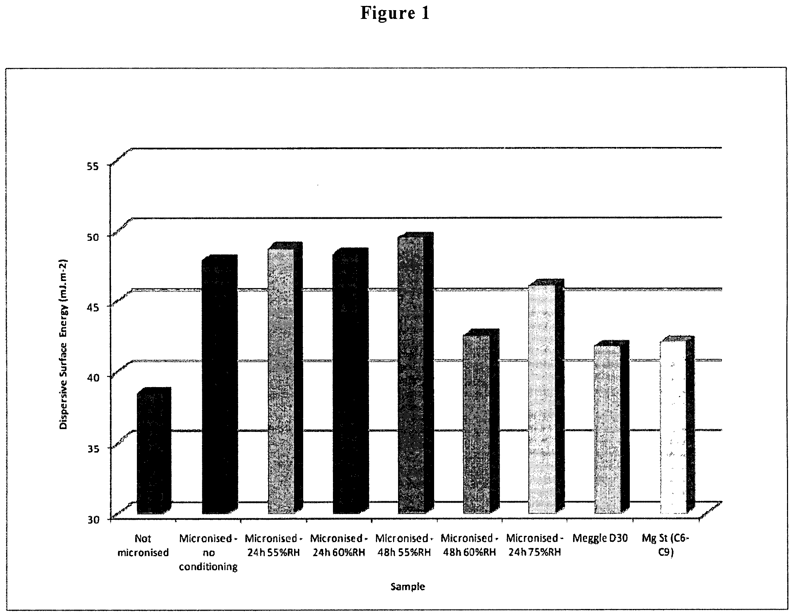

The results are reported in Figure 1.

Figure 1 shows the dispersive surface energy of each conditioned sample, along with the Meggle D30 and magnesium stearate (MgSt) references. The Figure illustrates that, relative to Maggle D30, each conditioned sample undergoes an increase in dispersive surface energy, demonstrating that the micronisation process induces and increase in the surface energy of lactose.

Inspection reveals that the dispersive surface energies of the processed Maggie D30 - MgSt Blends vary depending on their storage conditions. At 55% RH little change is observed in the dispersive surface energy of the micronized blends stored for 24 hours (48.7 mJm$^{-2}$) and 48 hours (49.5 mJm$^{-2}$). However, at 60% RH, a significant change is observed between the micronised blends stored for 24 and 48 hours (48.3 and 42.6 mJm$^{-2}$ respectively). The reduction in dispersive surface energy observed at 60% RH suggests that the samples have more readily adsorbed moisture from the surrounding environment. At this higher %RH the high energy sites present of the Maggle D30 - MgSt blends may have been quenched by moisture, possibly initiating the re-crystallisation of regions of amorphous lactose. This is supported by the similarity in dispersive surface energy of blends rested at 60% for 48 hours, and the dispersive surface energy of Meggle D30 reference (42.6 mJm$^{-2}$ vs. 41.8 mJm$^{-2}$).

Interestingly, the micronised blend rested for 24 hours at 75% RH exhibits a lower surface energy than the other blends rested for 24 hours (46.0 mJm$^{-2}$ vs 48.7 mJm$^{-2}$ and 48.3 mJm$^{-2}$). This further demonstrates that and increase in humidity is a prominent factor in reducing the dispersive energy if the micronised Meggle D30 - MgSt blends. However, the surface energy of the sample rested at 75% RH for 24 hours, is still greater than the blend rested at 60& RH for 48 hours, illustrating how a reduction in the dispersive surface energy of these blends appears to be dependent on both time and relative humidity.

The dispersive surface energy for lactose (41.8 mJm$^{-2}$) and magnesium stearate (42.1 mJm$^{-2}$) are both in good agreement with values reported in the literature (e.g. 41 mJm$^{-2}$ for lactose and 41 mJm$^{-2}$ for magnesium stearate).

Example 4 - Determination of the amorphous content

A spectroscopic approach involving H/D exchange and FT-Raman spectroscopy was used to probe the amorphous content of the micronised particles. The method exploits the fact that hydroxyl groups in amorphous lactose are susceptible to deuteration in an environment of deuterium oxide vapour, whereas crystalline lactose is not. The deuteration of the amorphous phase results in a shift in intensity from the OH-stretching region (3400 - 3150 cm$^{-1}$) to the OD-stretching region (2600 - 2300 cm$^{-1}$). The OD-stretching band can then be used as a direct indication of the level of amorphous content.

FT-Raman spectra were acquired from the samples before and after exposure to deuterium oxide vapour. Individual spectra were acquired for 5 minutes with laser power of 450 mW (at 1064 nm) and a resolution of 8 cm$^{-1}$. For each sample, before and after deuteration a total of ten spectra were acquired and averaged to account for any sample inhomogeneities.

Samples were exposed to a dynamic flow of deuterium oxide vapour (25%RH) generated and controlled by a Triton Humidity Generator (Triton.Technology, UK) for >12 hours. Dry, inert nitrogen was used as a carrier gas. After deuteration the samples were exposed to a flow of nitrogen gas for a further two hours in order to remove residual deuterium oxide.

Five samples of co-micronised particles were analysed (#1, #2, #3, #4 and #7) in comparison to not -conditioned and not-micronised reference samples.

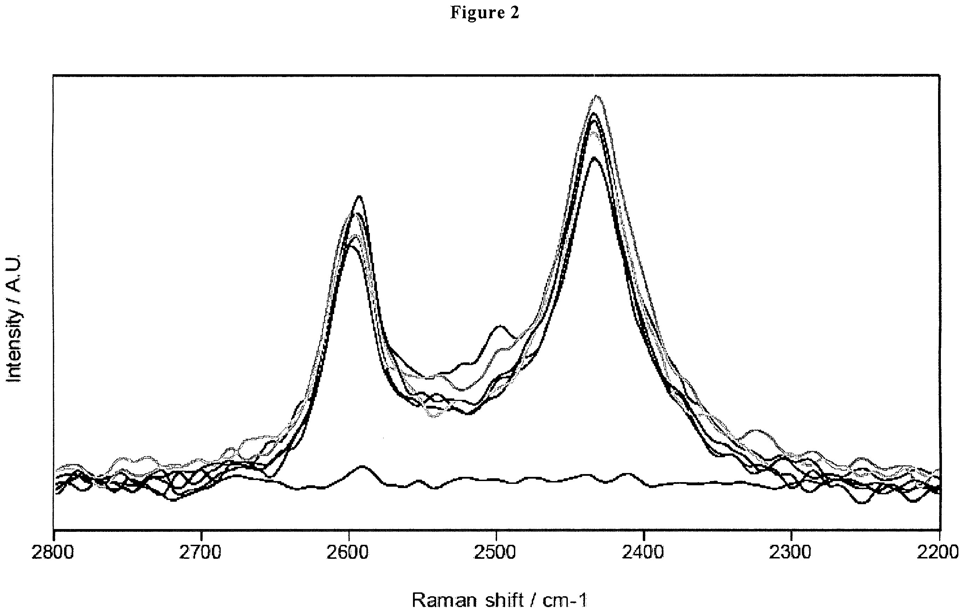

Figure 2 shows the OD stretching bands of the samples of co-micronised particles subjected to conditioning following exposure D$_2$O vapour (25% relative humidity for more than 12 hours).

The results indicate that all batches contain a significantly minor amount of amorphous material in conditioned samples than in not conditioned.

This suggests that the conditioning process employed has effectively re-crystallised a significant amount of amorphous material that was present in the pre-conditioned sample.

Example 5 - Preparation of the carrier

Each of the samples of co-micronised particles of Example 1 were mixed with fissured coarse particles of $\alpha$-lactose monohydrate having a mass diameter comprised between 212 - 355 micron, and obtained by sieving, in the ratio 90:10 percent by weight.

The mixing was carried out in a Turbula mixer for 4 hours.

The resulting mixtures of particles, termed hereinafter the CARRIER were analysed for particle size, with sieving system and flowability.

The particle size was determined by sieving.

The flow properties were tested according to the method described in the Eur. Ph.

Briefly, powder mixtures (about 110 g) were poured into a dry funnel equipped with an orifice of suitable diameter that is blocked by suitable mean. The bottom opening of the funnel is unblocked and the time needed for the entire sample to flow out of the funnel recorded. The flowability is expressed in seconds and tenths of seconds related to 100 g of sample.

While density and particle size were not affected by conditioning, flowability is decreased in the carriers comprising the conditioned co-micronised particles.

For said samples, the flow rate through a diameter of 4 mm turned out to be comprised between 136 and 134 s/100 g, while that of the carrier comprising the not-conditioned co-micronised particles turned out to be of about 142 s/100 g.

Example 6 - Preparation of the dry powder formulation

CARRIER particles comprising not-conditioned co-micronised particles, co-micronised particles sample #2 and sample #8 were used.

A portion of each CARRIER as obtained in Example 5 was mixed with micronised formoterol fumarate dihydrate (FF) in a Turbula mixer for 30 minutes at 32 r.p.m. and the resulting blend was forced through a sieve with mesh size of 0.3 mm (300 micron).

Micronised beclometasone dipropionate (BDP) and the remaining part of the CARRIER were blended in a Turbula mixer for 60 minutes at 32 r.p.m with the sieved mixture to obtain the final formulation.

The ratio of the active ingredients to 10 mg of CARRIER is 6 microg of FF dihydrate (theoretical delivered dose 4.5 microg) and 100 microg of BDP.

No agglomerates were observed during manufacturing.

The powder formulations were characterized in terms of the uniformity of distribution of the active ingredient and aerosol performances after loading it in the multidose dry powder inhaler described in WO 2004/012801.

The uniformity of distribution of the active ingredients was evaluated by withdrawing 20 samples from different parts of the blend and evaluated by HPLC.

The evaluation of the aerosol performance was carried out using the Andersen Cascade Impactor (Apparatus D) according to the conditions reported in the European Pharmacopeia 6th Ed 2008, par 2.9.18, pages 293-295.

After aerosolization of 10 doses, the ACI apparatus was disassembled and the amounts of drug deposited in the stages were recovered by washing with a solvent mixture and then quantified by High-Performance Liquid Chromatography (HPLC). The following parameters, were calculated: i) the delivered dose which is the amount of drug delivered from the device recovered in the impactor; ii) the fine particle dose (FPD) which is the amount of delivered dose recovered in the S3-AF stages having a particle size equal to or lower than 5.0 micron; iii) the fine particle fraction (FPF) which is the percentage of the fine particle dose; iv) the MMAD.

The results in terms of uniformity of distribution and aerosol performances (mean value ± S.D) are reported in Tables 4 and 5, respectively.

**Table 4 - Uniformity of distribution**

| Uniformity of distribution | not-conditioned | #2 | #8 |
|---|---|---|---|
| % FF (S.D.) | 97.9 (2.5%) | 101.6 (1.8%) | 103.0 (1.1%) |
| CV % | 2.6 | 1.8 | 1.1 |
| % BDP (S.D.) | 97.9 (2.1%) | 101.5 (1.5%) | 101.3 (1.1%) |
| CV % | 2.1 | 1.5 | 1.1 |

**Table 5 - Aerosol performances**

| Sample | not-conditioned | #2 | #8 |
|---|---|---|---|
| **FF** | | | |
| **Delivered Dose [ug]** | 3.77(±1.1) | 4.45(±0.3) | 4.58(±0.1) |
| **Fine Particle Dose [ug]** | 2.85(±1.0) | 2.73(±0.1) | 2.90(±0.08) |
| **Fine Particle Fraction [%]** | 59.36(±8.5) | 61.49(±0.7) | 63.32(±1.3) |
| **MMAD [um]** | 1.77 | 1.78 | 1.8 |
| **BDP** | | | |
| **Delivered Dose [ug]** | 78.81(±13.8) | 78.54(±2.7) | 78.19(±2.1) |
| **Fine Particle Dose [ug]** | 47.16(±8.5) | 46.49(±2.8) | 48.85(±1.1) |
| **Fine Particle Fraction [%]** | 59.82(±0.3) | 59.20(±1.5) | 62.49(±0.3) |
| **MMAD [um]** | 1.38 | 1.4 | 1.31 |

From the data of Table 4, it can be appreciated that the formulations prepared using the conditioned co-micronised particles show an increased uniformity of distribution of both active ingredients in comparison to that comprising the not-conditioned co-micronised particles.

From the data of Table 5, it can also be appreciated that the formulations prepared using the conditioned co-micronised particles provide a more accurate delivered dose of FF, the active ingredient present in a lower dose. Moreover, the formulations prepared using the conditioned co-micronised particles show a trend for improved respirable fraction for both the active ingredients.

**Claims**

1.  A process for preparing carrier particles for a dry powder formulation for inhalation comprising i) a fraction of co-micronized particles made of a mixture of an excipient and an additive, the mixture having a mass median diameter (MMD) lower than 20 micron; ii) a fraction of coarse excipient particles having a MMD equal to or higher than 80 micron, said process comprising the following steps:

    a) co-micronizing the excipient particles and additive particles;
    b) adding and mixing the obtained co-micronized particles with the coarse excipient particles;

    **characterized in that** the co-micronized particles of step a) are first conditioned by exposure to a relative humidity of 50-75% at 22 ± 2° C for a time comprised between 24 and 60 hours.

2.  The process according to claim 1, wherein the co-micronized particles are conditioned for a time of 48 hours.

3.  The process according to claim 1 or 2, wherein the additive is magnesium stearate.

4.  The process according to any one of claims 1 to 3, wherein the excipient is alpha-lactose monohydrate.

5.  The process according to any one of the preceding claims, wherein the mass diameter of the coarse excipient particles is comprised between 212 and 355 micron.

6.  A process for preparing a dry powder formulation for inhalation comprising the step of mixing the carrier particles according to any one of the preceding claims with one or more active ingredients.

7.  The process according to claim 6 wherein the active ingredient is selected from the group consisting of β2-adrenoceptor agonist, a corticosteroid, an anti-cholinergic alone or in any combination thereof.

8.  The process according to claim 7, wherein the active ingredient is a β2-adrenoceptor agonist selected from the group consisting of salbutamol, terbutaline, fenoterol, salmeterol, formoterol, indacaterol, vilanterol, and milveterol.

9.  The process according to claim 6, wherein the active ingredient is a corticosteroid selected from the group consisting of budesonide, fluticasone propionate, fluticasone furoate, mometasone furoate, beclomethasone dipropionate and ciclesonide.

10. The process according to claim 6, wherein the active ingredient is an anti-cholinergic bronchodilator selected from the group consisting of, ipratropium bromide, tiotropium bromide oxitropium bromide, and glycopyrronium bromide.

11. The process according to claim 7, wherein the β2-adrenoceptor agonist is fomoterol fumarate dihydrate, the corticosteroid is beclometasone dipropionate, and the anti-cholinergic is glycopyrronium bromide or tiotropium bromide.

**Patentansprüche**

1.  Verfahren zur Herstellung von Trägerpartikeln für eine Trockenpulverformulierung zur Inhalation, umfassend i) eine Fraktion co-mikronisierter Partikel, die aus einem Gemisch eines Exzipiens und eines Additivs hergestellt wurden, wobei das Gemisch einen massenmedianen Durchmesser (MMD) von kleiner als 20 Mikrometer hat; ii) eine Fraktion grober Exzipiens-Partikel, die einen MMD von gleich oder größer als 80 Mikrometer haben, wobei das Verfahren die folgenden Schritte umfasst:

    a) Co-mikronisieren der Exzipiens-Partikel und der Additiv-Partikel;
    b) Versetzen und Mischen der erhaltenen co-mikronisierten Partikel mit den groben Exzipiens-Partikeln;

    **dadurch gekennzeichnet, dass** die co-mikronisierten Partikel von Schritt a) zuerst durch Exposition gegenüber einer relativen Feuchtigkeit von 50-75% bei 22 ± 2°C für eine Zeit, die zwischen 24 und 60 Stunden liegt, konditioniert werden.

2.  Verfahren gemäß Anspruch 1, wobei die co-mikronisierten Partikel für eine Zeit von 48 Stunden konditioniert werden.

**3.** Verfahren gemäß Anspruch 1 oder 2, wobei das Additiv Magnesiumstearat ist.

**4.** Verfahren gemäß einem der Ansprüche 1 bis 3, wobei das Exzipiens alpha-Lactose-Monohydrat ist.

**5.** Verfahren gemäß einem der vorangehenden Ansprüche, wobei der Massendurchmesser der groben Exzipiens-Partikel zwischen 212 und 355 Mikrometer liegt.

**6.** Verfahren zur Herstellung einer Trockenpulverformulierung zur Inhalation, umfassend den Schritt des Mischens der Trägerpartikel gemäß einem der vorangehenden Ansprüche mit einem aktiven Ingrediens oder mehreren aktiven Ingredienzien.

**7.** Verfahren gemäß Anspruch 6, wobei das aktive Ingrediens aus der Gruppe, bestehend aus $\beta$2-Adrenoceptoragonist, einem Corticosteroid, einem Anticholinergikum alleine oder in einer beliebigen Kombination davon, ausgewählt wird.

**8.** Verfahren gemäß Anspruch 7, wobei das aktive Ingrediens ein $\beta$2-Adrenoceptoragonist, ausgewählt aus der Gruppe, bestehend aus Salbutamol, Terbutalin, Fenoterol, Salmeterol, Formoterol, Indacaterol, Vilanterol und Milveterol, ist.

**9.** Verfahren gemäß Anspruch 6, wobei das aktive Ingrediens ein Corticosteroid, ausgewählt aus der Gruppe, bestehend aus Budesonid, Fluticasonpropionat, Fluticasonfuroat, Mometasonfuroat, Beclomethasondipropionat und Ciclesonid, ist.

**10.** Verfahren gemäß Anspruch 6, wobei das aktive Ingrediens ein anticholinerger Bronchodilator, ausgewählt aus der Gruppe, bestehend aus Ipratropiumbromid, Tiotropiumbromid, Oxitropiumbromid und Glycopyrroniumbromid, ist.

**11.** Verfahren gemäß Anspruch 7, wobei der $\beta$2-Adrenoceptoragonist Fomoterolfumarat-Dihydrat ist, das Corticosteroid Beclometasondipropionat ist und das Anticholinergikum Glycopyrroniumbromid oder Tiotropiumbromid ist.

**Revendications**

**1.** Procédé de préparation de particules de support pour une formulation de poudre sèche pour inhalation comprenant i) une fraction de particules co-micronisées constituée d'un mélange d'un excipient et d'un additif, le mélange ayant un diamètre de masse médian (MMD) inférieur à 20 microns ; ii) une fraction de particules d'excipient grossières ayant un MMD supérieur ou égal à 80 microns, ledit procédé comprenant les étapes suivantes :

   a) co-micronisation des particules d'excipient et des particules d'additif ;
   b) ajout et mélange des particules co-micronisées obtenues avec les particules d'excipient grossières ;

   **caractérisé en ce que** les particules co-micronisées de l'étape a) sont d'abord conditionnées par exposition à une humidité relative de 50 à 75 % à 22 $\pm$ 2 °C pendant une durée comprise entre 24 et 60 heures.

**2.** Procédé selon la revendication 1, dans lequel les particules co-micronisées sont conditionnées pendant une durée de 48 heures.

**3.** Procédé selon la revendication 1 ou 2, dans lequel l'additif est le stéarate de magnésium.

**4.** Procédé selon l'une quelconque des revendications 1 à 3, dans lequel l'excipient est l'alpha-lactose monohydraté.

**5.** Procédé selon l'une quelconque des revendications précédentes, dans lequel le diamètre de masse des particules d'excipient grossières est compris entre 212 et 355 microns.

**6.** Procédé de préparation d'une formulation de poudre sèche pour inhalation comprenant l'étape de mélange des particules de support selon l'une quelconque des revendications précédentes avec un ou plusieurs principes actifs.

**7.** Procédé selon la revendication 6, dans lequel le principe actif est sélectionné dans le groupe constitué par un agoniste des récepteurs $\beta$2-adrénergiques, un corticostéroïde, un anticholinergique seul ou dans n'importe quelle combinaison de ceux-ci.

**8.** Procédé selon la revendication 7, dans lequel le principe actif est un agoniste des récepteurs $\beta$2-adrénergiques sélectionné dans le groupe constitué par le salbutamol, la terbutaline, le fénotérol, le salmétérol, le formotérol, l'indacatérol, le vilantérol et le milvétérol.

**9.** Procédé selon la revendication 6, dans lequel le principe actif est un corticostéroïde sélectionné dans le groupe constitué par le budésonide, le propionate de fluticasone, le furoate de fluticasone, le furoate de mométasone, le dipropionate de béclométhasone et le ciclésonide.

**10.** Procédé selon la revendication 6, dans lequel le principe actif est un bronchodilatateur anticholinergique sélectionné dans le groupe constitué par le bromure d'ipratropium, le bromure de tiotropium, le bromure d'oxitropium et le bromure de glycopyrronium.

**11.** Procédé selon la revendication 7, dans lequel l'agoniste des récepteurs $\beta$2-adrénergiques est le fumarate de fomotérol dihydraté, le corticostéroïde est le dipropionate de béclométasone et l'anti-cholinergique est le bromure de glycopyrronium ou le bromure de tiotropium.

Figure 1

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0178693 A
- WO 0178695 A
- US 200402022616 A
- WO 2010015324 A
- WO 2008006509 A
- WO 2009018909 A
- WO 9623485 A
- WO 2004012801 A

**Non-patent literature cited in the description**

- **GUCHARDI R et al.** *Int J Pharm,* 2008, vol. 348, 10-17
- **H. SUCKER ; P. FUCHS ; P. SPEISER.** Pharmaceutical Technology. Georg Thieme Verlag, 1991, 85
- **COLOMBO I et al.** *Il Farmaco,* 1984, vol. 39 (10), 328-341
- the European Pharmacopeia. 2008, 293-295